(19) Europäisches Patentamt
European Patent Office
Office européen des brevets

(11) EP 2 829 267 B1

(12) EUROPEAN PATENT SPECIFICATION

(45) Date of publication and mention of the grant of the patent:
**06.05.2020 Bulletin 2020/19**

(21) Application number: **13763714.6**

(22) Date of filing: **07.03.2013**

(51) Int Cl.:
***A61K 9/22*** (2006.01) ***A61K 31/7048*** (2006.01)
***A61K 47/38*** (2006.01) ***A61K 47/32*** (2006.01)
***A61P 25/06*** (2006.01) ***A61P 25/08*** (2006.01)
***A61P 3/10*** (2006.01) ***A61P 25/00*** (2006.01)
***A61P 25/24*** (2006.01) ***A61P 25/18*** (2006.01)
***A61P 9/12*** (2006.01)

(86) International application number:
**PCT/CN2013/072283**

(87) International publication number:
**WO 2013/139209 (26.09.2013 Gazette 2013/39)**

# (54) TOPIRAMATE SUSTAINED-RELEASE PHARMACEUTICAL COMPOSITION, METHOD FOR PREPARING SAME, AND USE THEREOF

PHARMAZEUTISCHE TOPIRAMATZUSAMMENSETZUNG MIT VERZÖGERTER FREISETZUNG, VERFAHREN ZUR HERSTELLUNG DAVON UND VERWENDUNG DAVON

COMPOSITION PHARMACEUTIQUE À LIBÉRATION PROLONGÉE À BASE DE TOPIRAMATE, SON PROCÉDÉ DE PRÉPARATION ET SON UTILISATION

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **23.03.2012 CN 201210080716**

(43) Date of publication of application:
**28.01.2015 Bulletin 2015/05**

(73) Proprietor: **Institute of Pharmacology and Toxicology Academy of Military Medical Sciences P.L.A. China**
**Beijing 100850 (CN)**

(72) Inventors:
- **LI, Song**
  **Beijing 100850 (CN)**
- **GAO, Chunsheng**
  **Beijing 100850 (CN)**
- **ZHONG, Wu**
  **Beijing 100850 (CN)**
- **WANG, Yuli**
  **Beijing 100850 (CN)**
- **YANG, Meiyan**
  **Beijing 100850 (CN)**
- **SHAN, Li**
  **Beijing 100850 (CN)**
- **ZHOU, Xinbo**
  **Beijing 100850 (CN)**
- **ZHENG, Zhibing**
  **Beijing 100850 (CN)**
- **WANG, Xiaokui**
  **Beijing 100850 (CN)**

(74) Representative: **Inspicos P/S**
**Kogle Allé 2**
**2970 Hørsholm (DK)**

(56) References cited:
**WO-A1-2007/102714 WO-A1-2011/095973**
**WO-A2-2005/079748 WO-A2-2011/107855**
**CN-A- 101 862 297 US-A1- 2011 287 103**

- **BOWEN P: "Particle Size Distribution Measurement from Millimeters to Nanometers and from Rods to Platelets", JOURNAL OF DISPERSION SCIENCE AND TECHNOLOGY, TAYLOR AND FRANCIS GROUP, NEW YORK, NY, US, vol. 23, no. 5, 1 January 2002 (2002-01-01), pages 631-662, XP009102859, ISSN: 0193-2691, DOI: 10.1081/DIS-120015368**

Note: Within nine months of the publication of the mention of the grant of the European patent in the European Patent Bulletin, any person may give notice to the European Patent Office of opposition to that patent, in accordance with the Implementing Regulations. Notice of opposition shall not be deemed to have been filed until the opposition fee has been paid. (Art. 99(1) European Patent Convention).

EP 2 829 267 B1

## Description

### Technical Field

The present invention pertains to medicine and chemical fields, and relates to sustained-release pharmaceutical composition of topiramate, its preparation method and uses. Specifically, the sustained-release pharmaceutical composition of topiramate is sustained-release pellet.

### Background Art

Topiramate (2,3,4,5-bis-O-(1-methylethylene)-β-D-fructopyranose sulfamate) (as shown in the following Formula 1) is a broad spectrum nerve therapeutic agent approved by FDA in 1995, and has been used in clinic for many years for treatment of some epileptic seizures and prevention of migraine headache (E. Faught, et al., (1996) Neurology 46:1684-1690), and many documents disclosed the good therapeutic effects of topiramate in treatment of diabetes (US7109174B2 and US6362220B1), dysneuria (US6908902B2), depression (US6627653B2), mental disorders (US6620819B2), headache (US6319903B1) and hypertension (US6201010B1).

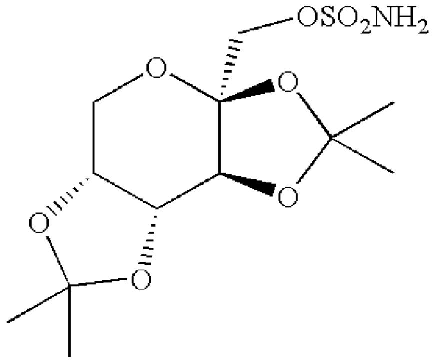

Formula 1

Topiramate is white crystalline powder, has bitter taste, is freely soluble in organic solvents such as acetone, chloroform, dimethylsulfoxide and ethanol, very freely soluble in alkaline solution having pH of 9-10 such as sodium hydroxide or sodium phosphate solutions, very slightly soluble in water (room temperature) with solubility of only about 9.8mg/mL, and its saturated solution has pH value of 6.3 (Physician's Desk Reference, 56.sup.th ed., pp. 2590-2595 (2002)).

Topiramate has linear pharmacokinetic features, can be rapidly and completely absorbed in vivo. After being orally taken in dose of 400mg by health volunteer, it can reach average plasma peak concentration (Cmax) within 2 h. Topiramate shows linear relation between blood concentration and dose in daily dose range of 100 mg to 800mg, and has a low clearance for oral administration (22-36 ml/min) and a long plasma half-life (19-25 h). In plasma concentration range of 0.5-250μm/ml, topiramate has a human plasma protein binding ratio of 15-41%, which decreases with the increase of plasma concentration.

At present, the dosage forms of topiramate used in clinic are normal tablets and capsules, in which the tablets have 4 specifications, i.e., 25mg, 50mg, 100mg, and 200mg; the capsules are sprinkle capsules and have 2 specifications, i.e., 15mg and 25mg, and since they have to be orally administrated for several times and dosages should be regulated, their administration is complicated and patients have poor compliance. More important, topiramate has a narrow therapeutic window, because the fluctuation of blood concentration usually results in some adverse reactions, which are mainly symptoms associated with central nervous system, such as ataxia, attention impairment, confusion, dizziness, fatigue, paresthesia, somnolence, and thinking abnormal, etc. (Physician's Desk Reference, 60th ed., pp 2538-2447(2006)).

Thus, in order to elevate compliance in patients and improve pharmaceutical efficiency, it is necessary to provide a sustained-release dosage form of topiramate that can reduce fluctuation of blood concentration and needs only one drug administration per day.

Oral sustained-release preparations, especially oral sustained-release pellets, have technical feature of "dose distribution", so that the drug is distributed more homogeneously in gastrointestinal tract, and absorbed more uniformly. In addition, the reduction of administration number renders drug-time curve relatively smooth, reduces occurrence rate of toxic and side effects, and significantly improve compliance in patients, so that they are very important in pharmaceutical

market and very popular in doctors and patients.

CN1988889A discloses a sustained-release preparation prepared by secondary granules, in which solid dispersion granules of topiramate are firstly prepared by melting method, then sustained-release granules are prepared by using sustained-release materials and the solid dispersion granules via one-step granulation or wet granulation methods, which has high production cost and complicated process.

CN102112126A discloses a sustained-release composition of low-dose topiramate which is used in combination with low-dose rapid-release phentermine for treatment of obesity, the sustained-release composition is prepared by firstly preparing topiramate drug-loading matrix cores (i.e., topiramate drug-loading cores) with 40% w/w of topiramate and 56.5% w/w of microcrystalline cellulose (AviceIPH102) via extrusion-spheronization method using 3.5% w/w of methyl cellulose (Methocel A15LV, MC) as a binding agent, then coating the topiramate drug-loading cores with 5.47% w/w of ethyl cellulose as sustained-release coating film material and 2.39% w/w of Povidone K30 (PVP K30) as a pore-forming agent, and finally forming topiramate pellets with controlled-release function.

WO2008061226A2 discloses sustained-release pellets of topiramate, which is prepared by performing drug-loading on surface of inert pellets such as sugar pellets with an aqueous dispersion containing 10-20% (w/w) topiramate and 0.5-4% HPMC or other binding agents via a fluidized bed coating method, then performing controlled-release coating on surface of the topiramate-loaded pellets, and further discloses that when inert pellets have a small particle diameter or pellets with high drug-loading rate are desired, high concentration of binding agent is necessary.

However, as for the above prepared sustained-release or controlled-release pellets (or pellets) of topiramate, the processes for preparing topiramate drug-loading pellet cores (or called as topiramate drug-loading matrix cores) all use a binding agent, such as HPMC or MC, etc., which means the increase of possibility of compatibility reaction with main drug topiramate, and in the meantime, the binding agent may influence dissolution state of the main drug and result in fluctuation of drug-release rate and effects on controlled-release. In addition, the adhesion degree of pellets would increase and the yield would decrease when a binding agent is added to solution during the process for loading drug on blank pellets.

Hence, it is extremely needed to provide a sustained-release preparation of topiramate which has desired drug release, high stability, and high yield, and can be readily prepared.

WO2011/107855 discloses the use of a binding agent in the drug layer, where either a "drug-binder solution" or a "drug-binder resin complex" is used in the preparation of a sustained-release pharmaceutical composition.

WO2005/079748 discloses the use of a binding agent in the drug layer of a sustained-release pharmaceutical preparation.

WO2011/095973 relates to a pharmaceutical composition for oral administration formulated for extended release of rasagiline.

WO2007/102714 disclosed a safe controlled-release preparation composition containing zolpidem which contain binding agent in the drug layer.

## Description of the Invention

With deep studying and inventive work, the inventors obtained a novel topiramate sustained-release composition (e.g., sustained- and controlled-release pellets), the topiramate sustained-release composition is free of a binding agent, and the inventors surprisingly found that the sustained-release pharmaceutical composition of topiramate has good sustained-release effects, high controllability, high stability, good repeatability, simple prescription, easy in operation and manufacture, and thus the following invention is provided.

The present invention provides a topiramate sustained-release pharmaceutical composition, which is a sustained-release pellet. The composition comprises: a) a blank pellet core; b) a drug layer, the drug layer is free of a binding agent; c) a sustained-release coating layer, in which the active drug layer is located on surface of the blank pellet core, and the sustained-release coating layer covers the external surface of the active drug layer. The schematic diagram of the sustained-release is shown in Fig.1.

In the composition of the present invention, the blank pellet cores refer to pellet cores without physiological activity, may include but not be limited to sugar pellets, microcrystalline pellets, starch pellets, or silicon dioxide pellets, etc., preferably sugar pellets. The blank pellet core has diameter of 150μm - 1500μm, preferably 300μm - 1000μm, more preferably 400μm - 850μm, most preferably 610μm - 750μm. The blank pellet cores can be commercially available in market, or prepared by conventional means such as extrusion spheronization method, fluidized bed method.

In the composition of the present invention, the active drug layer is free of a binding agent, in which the binding agent includes starch slurry, syrup, polyvinylpyrrolidone (povidone, PVP, such as PVP K30), methyl cellulose (MC), ethyl cellulose (EC), highly-substituted hydroxypropyl cellulose (HPC), hydroxypropyl methyl cellulose (HPMC), sodium carboxymethyl cellulose, gelatin, Arabic gum, etc. When a blank pellet core is loaded with drug without using a binding agent, the time for loading drug is short, and the adhesion degree of pellets is low.

In the composition of the present invention, the sustained-release coating layer comprises a sustained-release

coating material, including but not being limited to ethyl cellulose, Eudragit NE 30D, Eudragit RS 30D, or Eudragit RL30D, or a mixture thereof, preferably ethyl cellulose and Eudragit NE 30D, most preferably ethyl cellulose. The coating layer can further comprises a plasticizer, a pore-forming agent, an anti-sticking agent, a coloring agent, a light-screening agent, a flavoring agent, a sweetening agent, etc., in which the plasticizer includes but is not limited to glycerol, propylene glycol, polyethylene glycol, glycerol triacetate, triethyl citrate, phthalates or dibutyl sebate or a mixture thereof, preferably glycerol triacetate; the pore-forming agent includes but is not limited to polyethylene glycols, povidone, sucrose, salts, hydroxypropyl methyl cellulose, hydroxypropyl methyl cellulose or a mixture thereof, preferably povidone (PVP K30); the anti-sticking agent includes but is not limited to talc powder, magnesium stearate, aerosil or a mixture thereof, preferably talc powder; the light-screening agent includes but is not limited to titanium dioxide, etc.; the coloring agent includes but is not limited to iron oxide yellow, iron oxide red, coccinellin, lemon yellow, sunset yellow, indigo blue, etc.; the flavoring agent includes but is not limited to mint essence, lemon essence, orange essence, eucalyptol, caryophyllene alcohol, etc.; and the sweetening agent includes but is not limited to aspartame, vanillin, sorbitol, mannitol, artificial essences, or a mixture thereof.

In the present invention, the sustained-release coating has a weight increment (weight percentage of sustained-release coating film material to total composition, w/w) range that can be determined by tests, and generally, the weight increment range of the sustained-release coating is 2% - 30%, preferably 4% - 15%, more preferably 5% - 10%, relative to the total weight of the composition.

In the composition of the present invention, the active ingredient (topiramate) is in an amount of 10% - 50%, preferably 15% - 45%, more preferably 20% - 40%, relative to the total weight of the composition.

Further, the applicants found after a plenty of experiments that the above topiramate-carried pellet using the sustained-release coating layer comprising ethyl cellulose and PVP K30 in combination brought about unexpected good effects, compared with that of other sustained-release coating layers. That is, the topiramate pellet prepared with ethyl cellulose and PVP K30 as sustained-release coating layer material has better stability in drug release, which ensures consistency of drug release in different batches of samples, and the expected sustained-release effect can be achieved without heat treatment after coating. Thus, the coating process is simplified, and effects of heat treatment on drug release after coating are eliminated. The usage amount ratio of ethyl cellulose to PVP K30 is 1: 0.20 - 1: 0.45, preferably 1: 0.25 - 1: 0.40, especially preferably 1: 0.3 - 1: 0.35.

In a preferable embodiment of the present invention, the drug layer contains topiramate, the sustained-release coating layer uses ethyl cellulose as sustained-release coating material, PVP K30 as pore forming agent, and the usage amount ratio of ethyl cellulose to PVP K30 is 1: 0.20 - 1: 0.45.

In another preferable embodiment of the present invention, the drug layer contains topiramate, the sustained-release coating layer uses ethyl cellulose as sustained-release coating material, PVP K30 as pore forming agent, and the usage amount ratio of ethyl cellulose to PVP K30 is 1: 0.20 - 1: 0.45, and the range of weight increment of sustained-release coating is 5% - 15%.

In another preferable embodiment of the present invention, the blank pellet core is sugar pellet, the drug layer contains topiramate, the sustained-release coating layer uses ethyl cellulose as sustained-release coating material, PVP K30 as pore forming agent, the usage amount ratio of ethyl cellulose to PVP K30 is 1: 0.20 - 1: 0.45, and the range of weight increment of sustained-release coating is 5% - 15%.

In further another preferable embodiment of the present invention, the blank pellet core is sugar pellet having a particle diameter of 610μm - 750μm, the drug layer contains topiramate, the sustained-release coating layer uses ethyl cellulose as sustained-release coating material, PVP K30 as pore forming agent, the usage amount ratio of ethyl cellulose to PVP K30 is 1: 0.20 - 1: 0.45, and the range of weight increment of sustained-release coating is 5% - 10%.

In further another preferable embodiment of the present invention, the blank pellet core is sugar pellet having a particle diameter of 710μm - 850μm, the drug layer uses topiramate as active drug, the sustained-release coating layer uses ethyl cellulose as sustained-release coating material, PVP K30 as pore forming agent, the usage amount ratio of ethyl cellulose to PVP K30 is 1: 0.20 - 1: 0.45, and the range of weight increment of sustained-release coating is 5% - 8%.

In an particular embodiment of the present invention, the blank pellet core is sugar pellet having a particle diameter of 610μm - 750μm, the drug layer contains topiramate, the sustained-release coating layer uses ethyl cellulose as sustained-release coating material, PVP K30 as pore forming agent, the usage amount ratio of ethyl cellulose to PVP K30 is 1: 0.25 - 1: 0.35, and the range of weight increment of sustained-release coating is 6% - 8%.

In the present invention and the above embodiments, the drug layer contains topiramate, and further contains other pharmaceutically acceptable adjuvants, such as surfactants, disintegrating agents, flavoring agents, sweetening agents, anti-sticking agents, light-screening agents, etc. The surfactants include anionic surfactants, cationic surfactant, zwitterionic surfactants, and non-ionic surfactants, including but not being limited to sodium dodecyl sulfate, sodium hexadecanol sulfate, sodium octadecanol sulfate, sodium dodecylbenzene sulfonate, sodium dioctyl sulfosuccinate, sodium dihexyl sulfosuccinate, lecithin, sorbitan fatty acid esters, polyoxyethylene sorbitan fatty acid esters, polyoxyethylene fatty acid esters, polyoxyethylene fatty alcohol ethers, polymer of ethylene oxide and propylene oxide, polyoxyethylene 40 monostearate, polyoxyethylene 50 stearate, oxirane triblock copolymer, epoxypropane triblock copolymer, sorbitan monopalmitate (Span-40), sorbitan monostearate (Span-60), glyceryl monostearate, polyoxyethylene stearate, or mixtures thereof; the disintegrating agents include but are not limited to microcrystalline cellulose, low-substituted hydroxypropyl cellulose sodium, crosslinked polyvinylpyrrolidone, sodium carboxymethyl starch, pre-gelatinized starch, alginic acid, starch, effervescing disintegrants, or mixtures thereof; the anti-sticking agents include but are not limited to talc powder, magnesium stearate, aerosil, preferably talc powder; the light-screening agents include but are not limited to titanium dioxide, etc.; the flavoring agents include but are not limited to mint essence, lemon essence, orange essence, eucalyptol, caryophyllene alcohol, etc.; the sweetening agents include but are not limited to aspartame, vanillin, sorbitol, mannitol, artificial essences, etc.

The sustained-release pellet of topiramate of the present invention can bring about good therapeutic effect by once administration per 24 h, the in vivo blood concentration of drug is stable, peak concentration decreases significantly, and good sustained-release effect is achieved. The sustained-release pellet of topiramate of the present invention has in vitro release rate of: not greater than 35% within 1 h, between 30% and 60% within 4 h, between 60% and 90% within 8 h, and not less than 90% within in 16 h; preferably, not greater than 25% within 1 h, between 35% and 55% within 4 h, between 60% and 85% within 8 h, and not less than 90% within in 16 h; most preferably, not greater than 25% within 1 h, between 35% and 55% within 4 h, between 65% and 85% within 8 h, and not less than 90% within in 16 h.

The preferable conditions for determining the release rate in the present invention are in accordance with the first method (for sustained-release preparation or controlled-release preparation) of Release Rate Measurement (Appendix X D) of Part II of Chinese Pharmacopoeia, 2010 Edition, using apparatus as stated in the second method (slurry method) of Dissolution Rate Measurement (Appendix X C) of Part II of Chinese Pharmacopoeia, 2010 Edition, in which samples are taken and analyzed at different specified time points by using water (500 ml) as releasing media, at 37°C and rotation speed of 100 rpm.

On the other hand, the present invention further provides a method for preparing a sustained-release pharmaceutical composition of topiramate, the method comprising:

a) providing ingredients of drug layer to perform drug-loading and coating a blank pellet core;
b) subjecting the drug-loading pellet to sustained-release coating.

Preferably, the method for preparing a sustained-release pellet of topiramate in the present invention comprises the following steps:

a) providing topiramate and other adjuvants of drug layer, adding with a suitable amount of solvent for dissolution, and performing drug-loading and coating a blank pellet core to obtain a drug-loading pellet;
b) subjecting the drug-loading pellet to sustained-release coating.

More preferably, the method for preparing a sustained-release pellet of topiramate in the present invention comprises the following steps:

a) providing topiramate and other adjuvants of drug layer, adding with a suitable amount of solvent for dissolution, and performing drug-loading and coating a blank pellet core with the drug solution;
b) dissolving a sustained-release coating material and other adjuvants of sustained-release coating layer in a solvent, subjecting the drug-loading pellet to sustained-release coating.

Most preferably, the method for preparing a sustained-release pellet of topiramate in the present invention comprises the following steps:

a) providing topiramate and other adjuvants of drug layer, adding with a suitable amount of solvent, heating and dissolving under stirring, providing a blank pellet core and placing in a fluidized bed coating pan for one-step granulation, performing drug-loading and coating with the above drug solution under stirring;
b) dissolving a sustained-release coating material and other adjuvants of sustained-release coating layer in a solvent, heating and dissolving under stirring, mixing homogeneously, passing through a 100 mesh sieve, to obtain a sustained-release coating solution;
c) taking the drug-loading pellet, spraying the sustained-release coating solution on surface of the drug-loading pellet in a fluidized bed, to obtain a sustained-release pellet of topiramate.

The suitable solvent for the method of the present invention is water, ethanol, acetone, propylene glycol, chloroform or a mixture thereof, preferably a mixture of water and ethanol, for example, 50% ethanol water solution, 70% ethanol water solution, 95% ethanol water solution.

In the method for preparing sustained-release pellet of topiramate of the present invention, the active ingredient in drug layer is topiramate, the drug layer does not contain a binding agent, in which the binding agent refers to starch slurry, syrup, polyvinylpyrrolidone (povidone, PVP, such as PVP K30), methyl cellulose (MC), ethyl cellulose (EC), highly-substituted hydroxypropyl cellulose (HPC), hydroxypropyl methyl cellulose (HPMC), sodium carboxymethyl cellulose, gelatin, Arabic gum, etc. The drug layer can further comprises other pharmaceutically acceptable adjuvants, such as surfactants, disintegrating agents, flavoring agents, sweetening agents, anti-sticking agents, light-screening agents, etc. The surfactant include anionic surfactants, cationic surfactant, zwitterionic surfactants, and non-ionic surfactants, including but not being limited to sodium dodecyl sulfate, sodium hexadecanol sulfate, sodium octadecanol sulfate, sodium dodecylbenzene sulfonate, sodium dioctyl sulfosuccinate, sodium dihexyl sulfosuccinate, lecithin, sorbitan fatty acid esters, polyoxyethylene sorbitan fatty acid esters, polyoxyethylene fatty acid esters, polyoxyethylene fatty alcohol ethers, polymer of ethylene oxide and propylene oxide, polyoxyethylene 40 monostearate, polyoxyethylene 50 stearate, oxirane triblock copolymer, epoxypropane triblock copolymer, sorbitan monopalmitate (Span-40), sorbitan monostearate (Span-60), glyceryl monostearate, polyoxyethylene stearate, or mixtures thereof; the disintegrating agents include but are not limited to microcrystalline cellulose, low-substituted hydroxypropyl cellulose sodium, crosslinked polyvinylpyrrolidone, sodium carboxymethyl starch, pregelatinized starch, alginic acid, starch, effervescing disintegrants, or mixtures thereof; the anti-sticking agents include but are not limited to talc powder, magnesium stearate, aerosil, preferably talc powder; the light-screening agents include but are not limited to titanium dioxide, etc.; the flavoring agents include but are not limited to mint essence, lemon essence, orange essence, eucalyptol, caryophyllene alcohol, etc.; the sweetening agents include but are not limited to aspartame, vanillin, sorbitol, mannitol, artificial essences, etc.

In the method for preparing the composition of the present invention, the sustained-release coating material in the sustained-release coating layer includes but is not limited to ethyl cellulose, Eudragit NE 30D, Eudragit RS 30D, or Eudragit RL30D, preferably ethyl cellulose and Eudragit NE 30D, most preferably ethyl cellulose. The coating layer further comprises a plasticizer, a pore-forming agent, an anti-sticking agent, a coloring agent, a light-screening agent, a flavoring agent, a sweetening agent, etc., in which the plasticizer includes but is not limited to glycerol, propylene glycol, polyethylene glycol, glycerol triacetate, triethyl citrate, phthalates or dibutyl sebate, preferably glycerol triacetate; the pore-forming agent includes but is not limited to polyethylene glycols, povidone, sucrose, salts, hydroxypropyl methyl cellulose, hydroxypropyl methyl cellulose, etc. , preferably povidone (PVP K30); the anti-sticking agent includes but is not limited to talc powder, magnesium stearate, aerosil, preferably talc powder; the light-screening agent includes but is not limited to titanium dioxide, etc.; the coloring agent includes but is not limited to iron oxide yellow, iron oxide red, coccinellin, lemon yellow, sunset yellow, indigo blue, etc.; the flavoring agent includes but is not limited to mint essence, lemon essence, orange essence, eucalyptol, caryophyllene alcohol, etc.; and the sweetening agent includes but is not limited to aspartame, vanillin, sorbitol, mannitol, artificial essences, etc.

Preferably, the sustained-release coating layer of the sustained-release pellet of topiramate of the present invention contains ethyl cellulose and PVP K30.

In a specific embodiment of the present invention, the method for preparing pellet of topiramate comprises:

a) Topiramate is provided as main drug, dissolved with ethanol solution to prepare a solution with concentration of 20% (w/v) for drug-loading and coating. A blank pellet core is provided and placed in a fluidized bed coating pan for one step granulation, and the above drug solution is used for drug-loading and coating under stirring to obtain a drug-loading pellet core.

b) Ethyl cellulose as sustained-release coating material is dissolved in an ethanol solution to achieve a concentration of 3-8% (w/v), preferably 5-7% (w/v), and added with a suitable amount of specific pore-forming agent, PVP K30, then heated and dissolved under stirring, stirred homogenously, after passing through 100 mesh sieve, it is atomized and sprayed on the drug-loading pellet core with active drug layer of topiramate in a fluidized bed bottom-spraying coating pan to perform sustained-release coating.

The process parameters for drug-loading and sustained-release coating in the fluidized bed can be regulated according to practical situations, and preferable process parameters are as follows:

Drug-loading and coating - inlet air temperature is 50 - 70°C (to keep pan internal temperature at 40$\pm$2°C); inlet air pressure is 0.3 - 0.5bar; atomization pressure is 1.0 - 2.0bar; solution spray rate is 5 - 15g/min.

Sustained-release coating - inlet air temperature is 40 - 45°C (to keep pan internal temperature at 30 - 35°C); inlet air pressure is 0.3 - 0.5bar; atomization pressure is 1.0 - 2.0bar; solution spray rate is 3 - 12g/min.

The sustained-release pellet of topiramate of the composition of the present invention has a particle diameter of 150$\mu$m - 1500$\mu$m, preferably 300$\mu$m - 1000$\mu$m, more preferably 400$\mu$m - 850$\mu$m, most preferably 610$\mu$m - 750$\mu$m. The sustained-release pellet of topiramate of the composition of the present invention can be further processed to from other preparations, for example, can be loaded in capsules to form capsule preparation, or can be added with other pharmaceutically acceptable adjuvants and tableted to form tablets. It can also be combined with other active ingredients to form compound preparations.

The unit preparation of composition of the present invention can have a topiramate content of 1mg - 500mg, preferably 5mg - 300mg, more preferably 10mg - 250mg, most preferably 20mg - 100mg, optimally 23mg - 92mg.

In one embodiment of the present invention, the unit preparation contains 23mg of topiramate, and in another embodiment, the unit preparation contains 46mg of topiramate, and in further another embodiment, the unit preparation contains 92mg of topiramate.

The present invention further relates to a use of the sustained-release pharmaceutical composition of topiramate according to any one of items of the present invention in manufacture of a medicament for prophylaxis and/or treatment and/or adjunctive treatment of migraine, epilepsy, diabetes, dysneuria, depression, psychosis, headache, or hypertension.

Further disclosed herein is a method for prophylaxis and/or treatment and/or adjunctive treatment of migraine, epilepsy, diabetes, dysneuria, depression, psychosis, headache, or hypertension, comprising a step of administering an effective amount of the sustained-release pharmaceutical composition of topiramate according to any one of items of the present invention.

In the present invention the subject to be administered is a subject, such as a mammal, including but not being limited to: human, monkey, pig, cattle, goat, etc.

In the present invention, the term "effective amount" refers to a dose that can fulfill treatment, prophylaxis, alleviation and/or remission of the diseases or disorders of the present invention in the subject.

**Brief Description of the Drawings**

Fig.1: shows a schematic diagram of structure of topiramate pellet of the present invention.

Fig.2: shows release rate curves of the 3 batches of topiramate sustained-release coating pellets of Example 6 in water.

Fig.3: shows release rate curves of the first batch of topiramate sustained-release coating pellets of Example 6 in different release media.

Fig.4: shows release rate curves of the first batch of topiramate sustained-release coating pellets of Example 6 under different rotation speed conditions.

Fig.5: shows in vivo blood concentration curve of topiramate sustained-release coating pellets.

**Specific Models for Carrying Out the Invention**

The present invention is further illustrated with the following specific examples. It should be understood that the following examples are merely used to illustrate the present invention, but do not intend to limit the scope of the present invention. If specific conditions are not given in the examples, conventional conditions or conditions suggested by manufacturers were used. If the manufacturers of the used reagents or instruments were not given, they were all conventional products commercially available in markets.

In the following examples, unless specifically pointed out, the obtained parameters were all calculated according to the following formulations:

$$\text{Pellet drug-loading rate (\%)} = (W_{\text{total weight of pellets}} - W_{\text{blank pellet core weight}})/W_{\text{amount of bulk drug}} \times 100\%$$

$$\text{Weight increment of sustained-release coating (\%)} = (W_{\text{total weight of pellets after sustained-release coating}} - W_{\text{total weight of pellets before sustained-release coating}})/W_{\text{total weight of pellets after sustained-release coating}} \times 100\%$$

$$\text{Adhesion rate of pellet} = (W_{\text{total weight of pellets after coating}} - W_{\text{total weight of pellets without adhesion}})/W_{\text{total weight of pellets after coating}} \times 100\%$$

In the examples of the present invention, unless specifically pointed out, release rates of topiramate were all measured by the following method. According to the first method (for sustained-release preparation or controlled-release preparation) of Release Rate Measurement (Appendix X D) of Part II of Chinese Pharmacopoeia, 2010 Edition, the apparatus as stated in the second method (slurry method) of Dissolution Rate Measurement (Appendix X C) of Part II of Chinese Pharmacopoeia, 2010 Edition, was used to perform the measurement using water (500 ml) as releasing media, at 37°C and rotation speed of 100 rpm. Samples (5ml, supplemented with equivalent volume of media at the meantime) were taken at specified time points and filtrated, the subsequent filtrates were used as test solutions. High performance liquid chromatography (Appendix V D of Part II of Chinese Pharmacopoeia, 2010 Edition) was used, octylsilane-bonded silica gel was used as packing agent, column temperature was 35°C, 50% methanol was mobile phase, differential refractive detector was used, flow rate was 1.5ml per minute. 200 μl of test solution was taken, injected in liquid chromatograph, the peak area of topiramate as main drug was recorded; topiramate was separately taken as control sample and measured by the same method, and accumulative release percentages of drug at different time points were calculated by external standard method.

Example 1: Comparison of drug-loading and coating between drug-containing solutions with and without binding agent

Prescription:

| Prescription | Topiramate (g) | Binding agent (g) | | | | 50% ethanol (ml) |
|---|---|---|---|---|---|---|
| | | HPMC | PVP | HPC | Free of binding agent | |
| 1 | 230 | 6.9g | -- | -- | -- | 1150 |
| 2 | 230 | -- | 6.9g | -- | -- | 1150 |
| 3 | 230 | -- | -- | 6.9g | -- | 1150 |
| 4 | 230 | -- | -- | -- | -- | 1150 |

Preparation method:

4 Parts of topiramate raw material were weighed, 230g per part, separately added with suitable amount of 50% ethanol, stirred under heating at 40°C - 50°C for dissolution; then HPMC(E5), PVP K30 and HPC, each 6.9 g, were separately weighed, added in order to the first part, the second part, and the third part solutions, while the fourth part was free of binding agent; they were stirred and heated at 40°C - 50°C for dissolution, then added with 50% ethanol to reach 1150 ml to obtain drug-containing coating solutions with different binding agents.

500g of sucrose pellet cores (710 - 850μm) were placed in a fluidized bed bottom spray coating pan, the inlet air temperature was set as 55°C (to keep pan internal temperature at 40±2°C); inlet air pressure was 0.35bar; atomization pressure was 1.5bar; solution spray rate was 5 - 15g/min (regulated according to fluidization state at any time). The drug-containing coating solution was sprayed in manner of bottom spray on surface of blank pellet cores when the sucrose pellet cores were in fluidization state, after the end of drug-loading, the material was continuously fluidized at 45°C for 5 min to obtain drug-loading pellets with different binding agents, which were weighed, and results were shown in Table 1.

Table 1: Results of drug-loading and coating using drug-containing solutions with and without a binding agent

| Index | Prescription 1 | Prescription 2 | Prescription 3 | Prescription 4 |
|---|---|---|---|---|
| Adhesion degree (%) | 7.8 | 3.7 | 5.5 | 2.1 |
| Drug-loading time (min) | 71 | 62 | 68 | 54 |
| Drug-loading rate of pellet (%) | 94.2 | 93.7 | 95.1 | 95.4 |

The results showed that the sustained-release pharmaceutical composition of topiramate without binding agent in the present invention had short coating and drug-loading time, and high drug-loading rate.

Example 2: Results of drug-loading and coating using drug-containing coating solutions with different solvents

4 Parts of topiramate raw material were weighed, 230g per part, separately added with suitable amount of 50% ethanol, 70% ethanol, 95% ethanol, and anhydrous ethanol, stirred and heated at 40°C - 50°C for dissolution; then corresponding solvent was supplemented to reach 1150 ml to obtain drug-containing coating solutions with different solvents as dissolvent.

500g of sucrose pellet cores (710 - 850μm) were placed in a fluidized bed bottom spray coating pan, the inlet air temperature was set as 55°C (to keep pan internal temperature at 40±2°C); inlet air pressure was 0.35bar; atomization pressure was 1.5bar; solution spray rate was 5 - 15g/min (regulated according to fluidization state at any time). The drug-containing coating solution with different solvents as dissolvent was sprayed on surface of blank pellet cores in manner of bottom spray when the sucrose pellet cores were in fluidization state, after the end of drug-loading, the material was continuously fluidized at 45°C for 5 min to obtain drug-loading pellets with different solvents as dissolvent, which were weighed, and results were shown in Table 2.

Table 2: Drug-loading and coating results of drug-containing coating solutions with different solvents as dissolvent

| Index | 50% ethanol | 70% ethanol | 95% ethanol | Anhydrous ethanol |
|---|---|---|---|---|
| Adhesion degree (%) | 2.1 | 2.2 | 1.9 | 1.8 |
| Drug-loading time(min) | 54 | 50 | 48 | 44 |
| Drug-loading rate on pellet (%) | 95.4 | 94.5 | 91.6 | 90.4 |

Example 3: Comparison of release rates of sustained-release pellets of topiramate with blank pellet cores having different particle diameters

Prescription

1) Prescription of drug-loading pellet:

| Prescription | Topiramate (g) | Blank pellet core | |
|---|---|---|---|
| | | Range of particle diameter (μm) | Weight (g) |
| 5 | 230 | 300 - 400 | 500 |
| 6 | 230 | 500 - 610 | 500 |
| 7 | 230 | 610 - 750 | 500 |

2) Prescription of sustained-release coating layer:

| Prescription | Ethyl cellulose (g) | PVP K30 (g) |
|---|---|---|
| 5 | 50 | 16.5 |
| 6 | 40 | 13.2 |
| 7 | 30 | 10.0 |

Preparation method:

(1) 230g of topiramate raw material was weighed, added with a suitable amount of 50% ethanol, stirred and heated at 40-50°C for dissolution, added 50% ethanol to reach 1150 ml to obtain a drug-containing coating solution. 300μm - 400μm, 500μm - 610μm, 710μm - 850μm sucrose pellet cores were separately weighed, each 500 g, placed in a fluidized bed bottom spray coating pan, the inlet air temperature was set as 55°C (to keep pan internal temperature at 40±2°C); inlet air pressure was 0.35bar; atomization pressure was 1.5bar; solution spray rate was 5 - 15g/min (regulated according to fluidization state at any time). The drug-containing coating solution was sprayed

in manner of bottom spray on surface of blank pellet cores when the sucrose pellet cores were in fluidization state, after the end of drug-loading, the material was continuously fluidized at 45°C for 5 min to obtain drug-loading pellets of topiramate.
(2) The prescription amount of ethyl cellulose (EC) was weighed, added with a suitable amount of 95% ethanol for dissolution, then added with the prescription amount of PVP K30, dissolved to obtain a sustained-release coating solution.
(3) The above drug-loading pellets of topiramate with different particle diameters were weighed, each 500g, and separately placed in a fluidized bed bottom spray coating pan, the inlet air temperature was set as 40-45°C (to keep pan internal temperature at 30-35°C); inlet air pressure was 0.35bar; atomization pressure was 1.5bar; solution spray rate was 3-12 g/min. The sustained-release coating solutions of the 3 prescriptions were separately sprayed in manner of bottom spray on surfaces of drug-loading pellets with different particle diameters when the drug-loading pellets were in fluidization state, to obtain sustained-release pellets of topiramate with different particle diameters, in which the weight increments of sustained-release coating were 10.9%, 8.8% and 6.7%, respectively. According to calculation, the adhesion degrees of pellets were separately 2.2%, 2.1%, 1.8%.

The measurement results of drug release rates of the prepared sustained-release pellets of topiramate were shown in Table 3.

Table 3: Evaluation results of release rates of pellets with different particle diameters

| Prescription | Particle diameter of blank pellet cores | Release rate (%) | | | | | |
|---|---|---|---|---|---|---|---|
| | | 1h | 4h | 8h | 12h | 16h | 20h |
| 5 | 300 - 400μm | 21.6 | 55.5 | 88.1 | 98.5 | 100.4 | 101.2 |
| 6 | 500 - 610μm | 16.7 | 49.5 | 80.6 | 92.4 | 98.5 | 99.6 |
| 7 | 610 - 750μm | 19.4 | 56.8 | 85.4 | 96.2 | 99.8 | 100.4 |

Example 4: Comparison of release rates of sustained-release pellets of topiramate coated with different types of sustained-release materials

Prescription of sustained-release coating:

| Prescription | 8 | 9 | 10 |
|---|---|---|---|
| Drug-loading pellets | 500 g | 500 g | 500 g |
| Eudragit RS30D | 133 g(corresponding to 40g of dry resin) | -- | -- |
| Eudragit NE30D | -- | 167 g (corresponding to 50g of dry resin) | -- |
| Eudragit RL30D | -- | -- | 200 g(corresponding to 60g of dry resin) |
| Talc powder | 20 | 25 | 30 |
| Water | 246 | 309 | 370 |

Preparation method:

Aqueous dispersions of Eudragit RS30D, Eudragit NE 30D, Eudragit RL30D in prescription amounts were separately weighed, added with water in 1 time amount, stirred homogeneously; the talc powder in prescription amount was added to the residual water, homogenized with a high-shear homogenizer for 3 min, the obtained suspension was slowly poured into the above aqueous dispersions, stirred homogeneously, passed through 80 mesh sieve, to obtain sustained-release coating solutions.
500g of drug-loading pellets as prepared according to Prescription 4 of Example 1 was placed in a fluidized bed bottom spray coating pan, the inlet air temperature was set as 25-30°C (to keep pan internal temperature at 23-25°C); inlet air pressure was 0.35bar; atomization pressure was 1.5bar; solution spray rate was 3 - 5g/min. The above 3

sustained-release coating solutions were separately sprayed on surface of the drug-loading pellets in manner of bottom spray when the drug-loading pellets were in fluidization state, to obtain 3 kinds of sustained-release pellets respectively with Eudragit RS30D, Eudragit NE 30D, and Eudragit RL30D as sustained-release coating material, and the weight increments of sustained-release coating were separately 9.7%, 11.9%, and 13.9%. The obtained sustained-release pellets of topiramate were subjected to aging and heating treatment in a high temperature oven at 40°C for 24 h. According to calculation, the adhesion degrees of pellets were 2.8%, 2.7%, 3.0%, respectively.

The measurement results of drug release rates were shown in Table 4.

Table 4: Evaluation results of release rates of sustained-release pellets of topiramate with different sustained-release coating materials

| Prescription | Release rate (%) | | | | | |
|---|---|---|---|---|---|---|
| | 1h | 4h | 8h | 12h | 16h | 20h |
| 8 | 19.6 | 44.6 | 70.2 | 88.3 | 97.7 | 99.7 |
| 9 | 15.5 | 34.4 | 66.3 | 77.9 | 92.2 | 94.8 |
| 10 | 16.4 | 36.5 | 68.3 | 79.4 | 95.1 | 98.5 |

Example 5: Preparation of sustained-release pellets of topiramate

Prescription of sustained-release coating

| Prescription | 11 | 12 | 13 |
|---|---|---|---|
| EC | 30g | 30g | 30g |
| PVP K30 | 9g | 9.6g | 10.5g |

Preparation method:

According to the amount proportions of the above prescriptions, ethyl cellulose was dissolved with a suitable amount of 95% ethanol, then separately added with proportion amounts of PVP K30 and dissolved to obtain sustained-release coating solutions.

500g of drug-loading pellets as prepared with the drug-containing coating solution without a binding agent of Example 1 was placed in a fluidized bed bottom spray coating pan, the inlet air temperature was set as 40-45°C (to keep pan internal temperature at 30-35°C); inlet air pressure was 0.35bar; atomization pressure was 1.5bar; solution spray rate was 3 - 12g/min. The sustained-release coating solutions with different proportions of ethyl cellulose and PVP K30 were separately sprayed on surface of the drug-loading pellets in manner of bottom spray when the drug-loading pellets were in fluidization state, the weight increments of sustained-release coating were separately 6.56%, 6.65%, and 6.79%, so as to obtain sustained-release pellets of topiramate with different proportions of ethyl cellulose and PVP K30. According to calculation, the adhesion degrees of pellets were 2.3%, 2.4% and 2.1%, respectively.

The measurement results of drug release rate were shown in Table 5.

Table 5: Evaluation results of release rates of sustained-release pellet of topiramate as prepared according to the prescription 11-13

| Prescription | Release rate (%) | | | | | |
|---|---|---|---|---|---|---|
| | 1h | 4h | 8h | 12h | 16h | 20h |
| 11 | 17.4 | 36.6 | 64.3 | 82.5 | 90.7 | 95.5 |
| 12 | 21.8 | 47.6 | 76.4 | 91.3 | 95.7 | 97.8 |
| 13 | 24.2 | 51.4 | 83.8 | 99.7 | 99.6 | 100.2 |

Example 6: Experiment of process repeatability

(1) Preparation of topiramate drug-loading pellet cores without a binding agent

276 g of Topiramate raw material was weighed, added with a suitable amount of 70% ethanol, stirred under heating at 40-50°C, dissolved, added with 70% ethanol to reach 1380ml, to obtain a drug-containing coating solution.

600g of 710μm - 850μm sucrose pellet cores was weighed and placed in a fluidized bed bottom spray coating pan, the inlet air temperature was set as 55°C (to keep pan internal temperature at 40±2°C); inlet air pressure was 0.35bar; atomization pressure was 1.5bar; solution spray rate was 5 - 15g/min (regulated according to fluidization state at any time). The drug-containing coating solution was sprayed on surface of blank sucrose pellet cores in manner of bottom spray when the sucrose pellet cores were in fluidization state. After the end of drug-loading, the material was continuously fluidized at 45°C for 5 min to obtain topiramate drug-loading pellet cores without a binding agent, which were weighed, the total weight $W_{total}$ of the pellets after the end of drug-loading was recorded, and the drug-loading rate and product yield of the pellets were calculated and shown in Table 6.

Table 6: Results of process repeatability of topiramate drug-loading pellet cores without binding agent

| Sample batch | Production scale (preparation unit/batch) | Amount of the charged main drug (g/batch) | Amount of sucrose pellet cores (g/batch) | Amount of the produced drug-loading pellet cores (g/batch) | Drug-loading rate (%) | Product yield (%) |
|---|---|---|---|---|---|---|
| 1 | 12000 | 276 | 600 | 864 | 95.7 | 98.6 |
| 2 | 12000 | 276 | 600 | 862 | 94.9 | 98.4 |
| 3 | 12000 | 276 | 600 | 863 | 95.3 | 98.5 |
| Notation: the dose of topiramate of each preparation unit was expressed as 23mg; the product yield was calculated by dividing the amount of drug-loading pellet cores by the total amount of the charged raw materials and adjuvants. | | | | | | |

(2) Preparation of sustained-release coating pellet of topiramate

48g of Ethyl cellulose was weighed, added with a suitable amount of 95% ethanol, stirred under heating at 40°C - 50°C, dissolved, then added with about 16.2g of PVP K30, stirred under heating at 40°C - 50°C, dissolved, stirred homogenously, added with 95% ethanol to reach 1152ml, to obtain a sustained-release coating solution.

800g of drug-loading pellets as above prepared was placed in a fluidized bed bottom spray coating pan, the inlet air temperature was set as 40-45°C (to keep pan internal temperature at 30-35°C); inlet air pressure was 0.35bar; atomization pressure was 1.5bar; solution spray rate was 3 - 12g/min. The sustained-release coating solution was sprayed on surface of the drug-loading pellets in manner of bottom spray when the drug-loading pellets were in fluidization state, to obtain 3 batches of sustained-release pellets of topiramate, their weight increments of sustained-release coating were separately 6.87%, 6.98%, and 7.08%. According to calculation, the adhesion degrees of pellets were 2.1%, 2.0% and 2.1%, respectively. The results were shown in Fig.2 and Table 7.

Table 7: Experimental results of process repeatability of sustained-release coating pellets of topiramate

| Sample batch | Product amount (preparation unit/ batch) | Amount of drug-loading pellet cores, g/batch | Amount of ethyl cellulose, g/batch | Amount of PVP K30, g/batch | Sustained-release pellet of topiramate, g/batch | Product yield (%) | Release rate (%) | | | |
|---|---|---|---|---|---|---|---|---|---|---|
| | | | | | | | 1h | 4h | 8h | 16h |
| 1 | 10000 | 800 | 48 | 16.2 | 859 | 99.3 | 14.4 | 37.2 | 68.5 | 96.4 |
| 2 | 10000 | 800 | 48 | 16.2 | 860 | 99.5 | 15.1 | 38.6 | 69.5 | 96.8 |
| 3 | 10000 | 800 | 48 | 16.2 | 861 | 99.6 | 15.9 | 39.1 | 68.8 | 96.4 |

The results showed that the sustained-release pharmaceutical composition of topiramate (coating pellets) of the present invention had good process repeatability.

Example 7: Effects of different dissolution media on release rate of sustained-release pellet of topiramate

In order to verify whether acidic, basic solvent media would influence the release rate of the sustained-release pellets of the present invention, 0.1 mol/L HCl (pH1.2) was prepared as artificial gastric fluid, 0.2 ml/L phosphate buffer (pH6.8) was prepared as artificial intestinal fluid, and these media and water (500ml) were used as release media, rotation speed was 100 rpm, 37°C. Samples (5ml, supplemented with equivalent volume of media at the meantime) were taken at 1, 2, 4, 8, 12, 16, 20, 24h, filtrated, and the subsequent filtrates were used as test solutions. High performance liquid chromatography (Appendix V D of Part II of Chinese Pharmacopoeia, 2010 Edition) was used, octylsilane-bonded silica gel was used as packing agent, 50% methanol was mobile phase, differential refractive detector was used, flow rate was 1.5ml per minute. 200 μl of test solution was taken, injected in liquid chromatograph, the peak area of topiramate as main drug was recorded; topiramate was separately taken as control sample and measured by the same method, and accumulative release percentages of drug at different time points were calculated by external standard method. The release profile of sample of Batch 1 in Example 6 (2) in the above media were drawn, and the results were shown in Fig.3.

The results showed that the drug release profiles of the sustained-release pellet of topiramate in the artificial gastric fluid, water and the artificial intestinal fluid were substantially consistent (since topiramate was unstable in pH1.2 artificial gastric fluid and had degradation reaction, the release rate in the artificial gastric fluid in the present experiment was derived from the sum of main drug topiramate and degradation products), which suggested that the product could release drug consistently in different sites of gastrointestinal tract, so as to ensure stable pharmacological effects of topiramate as active ingredient.

Example 8: Effects of different rotation speeds on release rate of sustained-release pellet of topiramate

In order to verify whether gastrointestinal motility would influence the release rate of the sustained-release pellets of the present invention, rotation speed was set as 50 rpm, 75 rpm and 100 rpm, respectively, and water (500ml) were used as release media, 37°C. Samples (5ml, supplemented with equivalent volume of media at the meantime) were taken at 1, 2, 4, 8, 12, 16, 20, 24h, filtrated, and the subsequent filtrates were used as test solutions. High performance liquid chromatography (Appendix V D of Part II of Chinese Pharmacopoeia, 2010 Edition) was used, octylsilane-bonded silica gel was used as packing agent, 50% methanol was mobile phase, differential refractive detector was used, flow rate was 1.5ml per minute. 200 μl of test solution was taken, injected in liquid chromatograph, the peak area of topiramate as main drug was recorded; topiramate was separately taken as control sample and measured by the same method, and accumulative release percentages of drug at different time points were calculated by external standard method. The release profile of sample of Batch 1 in Example 6 (2) under the above different rotation speeds were drawn, and the results were shown in Fig.4.

The results showed that the drug release profiles of the sustained-release pellet of topiramate under rotation speed ranging 50-100 rpm were substantially consistent, which suggested that the product could release drug consistently under different situations of gastrointestinal motility, so as to ensure stable pharmacological effects of topiramate as active ingredient.

Example 9: Studying on drug release consistency (1)

The prescriptions 8-10 were repeated 3 times according to the method of Example 4, and their drug release consistency was considered. The results were shown in Table 8.

Table 8: Results of drug release consistency of sustained-release pellet of topiramate (mean ± standard deviation)

| Prescription | Drug release rate at different time points (%) (mean ± standard deviation) | | | | |
|---|---|---|---|---|---|
| | 1h | 4h | 8h | 16h | 20h |
| Prescription 8 | 19.6±8.7 | 51.2±8.3 | 75.2±9.8 | 93.3±9.1 | 95.7±5.2 |
| Prescription 9 | 15.1±7.1 | 38.8±7.4 | 68.7±8.7 | 92.9±9.8 | 95.3±4.1 |
| Prescription 10 | 14.4±8.8 | 36.5±8.3 | 65.3±9.6 | 92.4±8.1 | 95.1±5.2 |
| Example 6 | 14.4±2.5 | 37.2±1.8 | 68.5±1.4 | 96.4±1.2 | 100.2±0.9 |

The results showed that the sustained-release pharmaceutical composition of topiramate (coating pellets) of the present invention had good drug release consistency.

Example 10: Studying on drug release consistency (2)

Prescription:

Drug layer

| Prescription | Topiramate (g) | Sodium dodecyl sulfate (g) | Tween 80 (g) | Talc powder (g) |
|---|---|---|---|---|
| 14 | 230 | 3.45 | -- | -- |
| 15 | 230 | -- | 6.90 | -- |
| 16 | 230 | -- | -- | 11.5 |

Sustained release layer

| Name | Amount |
|---|---|
| EC (g) | 30 |
| PVP K30 (g) | 10.0 |
| Aerosil (g) | 3.0 |
| 95% ethanol (ml) | 720 |

Preparation method:

(1) The ingredients of drug layer in the above prescription amounts were weighed, added with a suitable amount of 50% ethanol, stirred under heating at 40°C-50°C, dissolved (prescriptions 14 and 15) or suspended (prescription 16), added with 50% ethanol to reach 1150ml, to obtain drug-containing coating solutions (prescriptions 14, 15) or suspension (prescription 16).
500g of 610μm - 750μm sucrose pellet cores was weighed and placed in a fluidized bed bottom spray coating pan, the inlet air temperature was set as 55°C (to keep pan internal temperature at 40±2°C); inlet air pressure was 0.35bar; atomization pressure was 1.5bar; solution spray rate was 5 - 15g/min (regulated according to fluidization state at any time). The drug-containing coating solutions or suspension were sprayed on surface of blank pellet cores in manner of bottom spray when the sucrose pellet cores were in fluidization state. After the end of drug-loading, the material was continuously fluidized at 45°C for 5 min to obtain topiramate drug-loading pellets.
(2) Ethyl cellulose (EC) in prescription amount was weighed, added with 95% ethanol, stirred under heating at 40°C - 50°C, dissolved, then added with the prescription amount of PVP K30, stirred under heating at 40°C - 50°C, dissolved, stirred homogeneously, added with the prescription amount of aerosil, added 95% ethanol to the prescription amount, stirred, to obtain sustained-release coating solution.

500g of the above topiramate drug-loading pellet cores was separately weighed and placed in a fluidized bed bottom spray coating pan, the inlet air temperature was set as 40-45°C (to keep pan internal temperature at 30-35°C); inlet air pressure was 0.35bar; atomization pressure was 1.5bar; solution spray rate was 3 - 12g/min. The sustained-release coating solutions of the 3 prescriptions were sprayed on surface of drug-loading pellets with different corresponding particle diameters in manner of bottom spray when the drug-loading pellets were in fluidization state, to obtain sustained-release pellets of topiramate with different particle diameters, the weight increment of coating was 6%. Via calculation, the adhesion degrees of pellets were 1.9, 2.0, 1.7%, respectively. The measurement results of release rates were shown in Table 9.

Table 9: Measurement results of drug release rates of prescriptions 14-16

| Prescription | Release rate (%) | | | | |
|---|---|---|---|---|---|
| | 1h | 4h | 8h | 16h | 20h |
| Prescription 14 | 9.60 | 39.6 | 66.3 | 95.7 | 97.1 |
| Prescription 15 | 9.4 | 36.6 | 65.3 | 96.8 | 98.8 |
| Prescription 16 | 9.2 | 37.8 | 68.2 | 96.9 | 99.7 |

The results showed that the sustained-release pharmaceutical composition of topiramate (coating pellets) of the present invention good drug release consistency.

Example 11: Preparation of sustained-release pellets of topiramate

500 g of the topiramate drug-loading pellet cores prepared according to Prescription 1 in Example 1, which drug layer contained binding agent HPMC, was weighed, and placed in a fluidized bed bottom spray coating pan. 720ml of 95% ethanol was used to prepare a sustained-release coating solution containing ethyl cellulose and PVP K30 respectively in amount of 30g and 10g. The inlet air temperature was set as 40-45°C (to keep pan internal temperature at 30-35°C); inlet air pressure was 0.35bar; atomization pressure was 1.5bar; solution spray rate was 3 - 12g/min. The sustained-release coating solution was sprayed on surface of drug-loading pellets in manner of bottom spray when the drug-loading pellets were in fluidization state, to obtain sustained-release pellets of topiramate with a drug layer containing binding agent HPMC, the weight increment of coating was 6.86.

Example 12: Preparation of sustained-release pellets of topiramate

Prescription

| Name of ingredients | Dosage per 10000 preparation units (g) | Dosage ratio (%)(w/w) |
|---|---|---|
| Topiramate | 230 | 36.85 |
| Microcrystalline cellulose (Avicel® PH102) | 324.9 | 52.05 |
| Methyl cellulose (Methocel™ A15LV) | 20.1 | 3.22 |
| Ethyl cellulose | 34.14 | 5.47 |
| Povidone (Povidone K30) | 14.92 | 2.39 |

Preparation method:

Topiramate as main drug and microcrystalline cellulose as filling agent in the prescription amounts passed through sieve and mixed homogeneously; 70% ethanol was used to dissolve methyl cellulose (Methocel™A15LV) to obtain a solution as binding agent with a suitable concentration, and then used to form soft material; the soft material was placed in an extruder using a certain mesh sieve and at a extrusion rate to extrude rod like granules; the extruded granules were placed in a spheronizator and spheronized under certain spheronization speed for 3-5 min, the obtained pellets were dried in 40°C oven for 2 h to obtain topiramate drug-loading pellet cores.

The prescription amounts of ethyl cellulose and Povidone (Povidone K30) were weighed, added with 820ml of 95% ethanol, stirred and dissolved to form a sustained-release coating solution. The above prepared topiramate drug-loading pellet cores were placed in a fluidized bed bottom spray coating pan, the inlet air temperature was set as 40-45°C (to keep pan internal temperature at 30-35°C); inlet air pressure was 0.35bar; atomization pressure was 1.5bar; solution spray rate was 1 - 3g/min. The sustained-release coating solutions was sprayed on surface of drug-loading pellet cores when the drug-loading pellets were in fluidization state, to obtain sustained-release pellets of topiramate, the weight increment of coating was 6.68%.

Example 13: Studying on stability of topiramate pellets

The sustained-release pellets of topiramate prepared in Example 11 and Example 12, and the first Batch of

sustained-release pellets of topiramate prepared in Example 6 were separately placed nakedly in sealed dryer with saturated NaCl solution, then the dryer was placed in high temperature 60°C oven, acceleration conditions of high temperature and high humidity (60°C, RH75%) were set in the meantime, and samples were taken on $0^{th}$, $5^{th}$ and $10^{th}$ day.

The content of pellet sample was poured out, placed in 10mL volumetric flask, dissolved with a suitable amount of methanol under ultrasonic waves, then diluted with water in 5 times volume to reach scale, so that the concentration of main drug was about 5mg/ml, 0.45μm organic microfiltration membrane was used for filtration, primary filtrate was discarded, the subsequent filtrate were used as test solutions. High performance liquid chromatography (Appendix V D of Part II of Chinese Pharmacopoeia, 2010 Edition) was used, octylsilane-bonded silica gel was used as packing agent, column temperature was 35°C, 40% methanol was mobile phase, differential refractive detector was used, flow rate was 1.5ml per minute. 200 μl of the test solution was taken, injected in liquid chromatograph, chromatogram was recorded until 3 times the time period of main peak retention time, if the test solution had peaks of impurities, the total content of impurities was calculated by peak area normalization method, and the results were shown in Table 10. It could be seen that the sustained-release composition of topiramate (the coating type drug-loading pellet cores in which the topiramate drug layer was free of binding agent, sample of Example 6) as disclosed in the present invention had stability superior to the matrix type drug-loading pellet cores (sample of Example 12) and the coating type drug-loading pellet cores (sample of Example 11) which all had topiramate drug layer containing a binding agent.

Table 10: Results of stability of sustained-release pellets of topiramate

| Sample of stability | Degradation products (relevant substances) (%) | | |
|---|---|---|---|
| | $0^{th}$ day | $5^{th}$ day | $10^{th}$ day |
| Sample of Example 11 | 0.15 | 0.37 | 0.55 |
| Sample of Example 12 | 0.15 | 0.38 | 0.57 |
| Sample of Example 6 | 0.15 | 0.21 | 0.28 |

The results showed that the sustained-release pharmaceutical composition of topiramate of the present invention had good stability.

Example 14: Studying on in vivo pharmacokinetics of sustained-release pellet of topiramate

Test samples: the topiramate drug-loading pellet core (rapid-release pellet) as prepared in Example 6 was used as reference preparation, and the sustained-release pellet of topiramate as prepared in Example 6 was used as test preparation. Administration dose was all 23 mg expressed as topiramate (main drug).

Test subjects: 6 Beagles, half male and half female, the body weight of Beagles was $8.97 \pm 1.05$ kg.

Dosage regimen: 6 Beagles were subjected to double cycle random crossover test design, separately orally administrated once with equivalent dose of the test preparation containing 23mg of topiramate as main drug and the reference preparation containing 23mg of topiramate as main drug, an wash-out period with interval time of 15 days was set between the two cycles. Blood samples (2mL) were separated taken from leg veins of the Beagles at 0.5, 1, 2, 3, 4, 6, 8, 10, 12, 16, 24, 36, 48h after administration, placed in negative pressure glass tubes treated with heparin sodium, centrifuged at 4000 r/min for 10 min to separate plasma, the plasma was removed to 1mL EP tube, labeled with test number, random number of Beagle and blood sampling time, the blood samples were kept at -20°C for treatment and analysis.

Plasma sample treatment: 100μL of plasma of Beagle after administration was taken, placed in 1.5 ml centrifuge tube, added with 20μl of water, added with 20μl of internal standard solution (500ng/ml Nimesulide solution), added with 0.5ml of methanol as precipitator, subjected to eddy for 3 min, centrifuged for 10 min (9500 rpm), supernatants (20μl) were separately sucked up, and analyzed with LC/MS/MS under the following chromatography conditions, and chromatograms were recorded.

Chromatography conditions: analysis column was Zorbax C8, 5μm particle size, 150 x 4.6 mm I.D., Agilent Company of US; pre-column was C18 protection column, 4 x 3.0 mm I.D., Phenomenex Company, USA; column temperature was 25°C; mobile phase was methanol-0.5 mM ammonium acetate (75:25, v/v); flow rate was 0.5 mL/min; internal standard was Nimesulide (500 ng/mL).

Mass spectrometric conditions: API 3000 type tandem quadrupole mass spectrometer. Ion source was atmospheric chemical ion source (Turbo lonspray source); detection was performed in negative ion manner; ejection voltage was - 4200 V; source temperature was 450°C; nebulizer gas (NEB) was 8; curtain gas (CUR) was 11; collision gas (CAD) was 5; scanning manner was multiple reaction monitoring (MRM), the ion reactions for quantitative analysis were separately: m/z 338 → m/z 78 (topiramater, CE - 55 V), m/z 307 → m/z 229 (internal standard nimesulide, CE - 20 V);

scanning time was 150 msec.

Pharmacokinetic data treatment: blood concentration data were analyzed with DAS 2.0 analytic software.

Results of measurement: after Beagles were orally administered with equivalent dose (23mg) of reference preparation (first batch of topiramate drug-loading pellet cores of Example 6) and test preparation (first batch of sustained-release pellet of topiramate of Example 6), average blood concentrations (μg/ml) at different time points were shown in Fig.5, and main pharmacokinetic parameters were shown in Table 11.

Table 11: Main pharmacokinetic parameters

| Test sample | $T_{max}$ (h) | $C_{max}$ ($\mu g \cdot ml^{-1}$) | $AUC_{0\rightarrow Tn}$ ($h \cdot \mu g \cdot ml^{-1}$) | Relative bioavailability (%) |
|---|---|---|---|---|
| Reference preparation | 2.33±0.47 | 1.11 ± 0.13 | 9.39 ± 3.23 | 100.00% |
| Test preparation | 7.33 ± 0.94 | 0.55 ± 0.07 | 8.72 ± 2.67 | 92.87% |

The results of Fig.5 and Table 11 showed that the Beagles orally administered with the sustained-release composition of topiramate as provided by the present invention (first batch of sustained-release pellets of topiramate of Example 6, containing 23mg of topiramate) showed significantly extended Tmax, significantly decreased Cmax, in comparison with the rapid-release topiramate drug-loading pellet cores (reference preparation, first batch of topiramate pellet cores of Example 1, containing 23 mg of topiramate), and more important, the relative bioavailability of the test preparation was 92.87% of that of the reference preparation. This indicated that the topiramate sustained-release composition as provided by the present invention had biologically equivalent to the reference preparation, and showed significant profiles of sustained-release preparation, that was, the peak concentration decreased significantly, and the action time was significantly extended.

Although the specific models of the present invention have been described in details, those skilled in the art would understand that these details can be modified or changed according to all teachings of disclosures in the art

**Claims**

1. A sustained-release pharmaceutical composition of topiramate, which is a topiramate sustained-release pellet, and the topiramate sustained-release pellet comprises a blank pellet core, a drug layer, and a sustained-release coating layer, in which the drug layer is free of binding agent.

2. The sustained-release pharmaceutical composition of topiramate according to claim 1, wherein topiramate is present in an amount of 10% - 50% relative to total weight of the composition.

3. The sustained-release pharmaceutical composition of topiramate according to claim 1, wherein topiramate is present in an amount of 15% - 45% relative to total weight of the composition.

4. The sustained-release pharmaceutical composition of topiramate according to claim 1, wherein topiramate is present in an amount of 20% - 40% relative to total weight of the composition.

5. The sustained-release pharmaceutical composition of topiramate according to claim 1, wherein the blank pellet core has a particle diameter of 150 μm - 1500 μm.

6. The sustained-release pharmaceutical composition of topiramate according to claim 1, wherein the blank pellet core has a particle diameter of 300 μm - 1000 μm.

7. The sustained-release pharmaceutical composition of topiramate according to claim 1, wherein the blank pellet core has a particle diameter of 400 μm - 850 μm.

8. The sustained-release pharmaceutical composition of topiramate according to claim 1, wherein the blank pellet core has a particle diameter of 610 μm - 750 μm.

9. The sustained-release pharmaceutical composition of topiramate according to claim 1, wherein the sustained-release coating layer has a weight increment range of 2% - 30%.

10. The sustained-release pharmaceutical composition of topiramate according to claim 1, wherein the sustained-release

coating layer has a weight increment range of 4% - 15%.

**11.** The sustained-release pharmaceutical composition of topiramate according to claim 1, wherein the sustained-release coating layer has a weight increment range of 5% - 10%.

**12.** The sustained-release pharmaceutical composition of topiramate according to claim 1, wherein the sustained-release coating layer comprises a sustained-release coating material, which is one or more selected from a group consisting of ethyl cellulose, Eudragit NE 30D, Eudragit RS 30D, Eudragit RL30D.

**13.** The sustained-release pharmaceutical composition of topiramate according to claim 1 or 12, wherein the sustained-release coating layer comprises ethyl cellulose and PVPK30.

**14.** The sustained-release pharmaceutical composition of topiramate according to claim 13, wherein the usage amount ratio of ethyl cellulose to PVP K30 is 1:0.20 - 1:0.45.

**15.** The sustained-release pharmaceutical composition of topiramate according to claim 13, wherein the usage amount ratio of ethyl cellulose to PVP K30 is 1:0.25 - 1:0.40.

**16.** The sustained-release pharmaceutical composition of topiramate according to claim 13, wherein the usage amount ratio of ethyl cellulose to PVP K30 is 1:0.3 - 1:0.35.

**17.** A method for preparing the sustained-release pharmaceutical composition of topiramate of any one of claims 1-17, comprising the following steps:

a) providing an active drug of drug layer to perform drug-loading and coating a blank pellet core to obtain a drug-loading pellet;
b) subjecting the drug-loading pellet to sustained-release coating.

**18.** The method according to claim 17, comprising the following steps:

a) providing topiramate and other adjuvants of drug layer, adding with a suitable amount of solvent for dissolution, and performing drug-loading and coating a blank pellet core to obtain a drug-loading pellet;
b) subjecting the drug-loading pellet to sustained-release coating;

**19.** The method according to claim 17, comprising the following steps:

a) providing topiramate and other adjuvants of drug layer, adding with a suitable amount of solvent for dissolution, and performing drug-loading and coating a blank pellet core with the drug solution, to obtain a drug-loading pellet;
b) dissolving a sustained-release coating material and other adjuvants of sustained-release coating layer in a solvent, subjecting the drug-loading pellet to sustained-release coating;

**20.** The method according to claim 17, comprising the following steps:

a) providing topiramate and other adjuvants of drug layer, adding with a suitable amount of solvent, heating and dissolving under stirring, providing a blank pellet core and placing in a fluidized bed coating pan for one-step granulation, performing drug-loading and coating with the above drug solution under stirring, to obtain a drug-loading pellet;
b) dissolving a sustained-release coating material and other adjuvants of sustained-release coating layer in a solvent, heating and dissolving under stirring, mixing homogeneously, passing through a 100 mesh sieve, to obtain a sustained-release coating solution;
c) taking the drug-loading pellet, spraying the sustained-release coating solution on surface of the drug-loading pellet in a fluidized bed, to obtain a sustained-release pellet of topiramate.

**21.** Use of the sustained-release pharmaceutical composition of topiramate according to any one of claims 1-16 in manufacture of a medicament for prophylaxis and/or treatment and/or adjunctive treatment of migraine, epilepsy, diabetes, dysneuria, depression, psychosis, headache, or hypertension.

**22.** A sustained-release pharmaceutical composition according to any one of claims 1-16 for use in the prophylaxis

and/or treatment and/or adjunctive treatment of migraine, epilepsy, diabetes, dysneuria, depression, psychosis, headache, or hypertension.

## Patentansprüche

1. Pharmazeutische Zusammensetzung mit verzögerter Freisetzung von Topiramat, bei der es sich um ein Topiramat-Pellet mit verzögerter Freisetzung handelt und das Topiramat-Pellet mit verzögerter Freisetzung einen leeren Pelletkern, eine Wirkstoffschicht und eine Beschichtungsschicht mit verzögerter Freisetzung umfasst, wobei die Wirkstoffschicht frei von Bindemittel ist.

2. Pharmazeutische Zusammensetzung mit verzögerter Freisetzung von Topiramat nach Anspruch 1, wobei Topiramat in einer Menge von 10 % bis 50 % bezogen auf das Gesamtgewicht der Zusammensetzung vorliegt.

3. Pharmazeutische Zusammensetzung mit verzögerter Freisetzung von Topiramat nach Anspruch 1, wobei Topiramat in einer Menge von 15 % bis 45 % bezogen auf das Gesamtgewicht der Zusammensetzung vorliegt.

4. Pharmazeutische Zusammensetzung mit verzögerter Freisetzung von Topiramat nach Anspruch 1, wobei Topiramat in einer Menge von 20 % bis 40 % bezogen auf das Gesamtgewicht der Zusammensetzung vorliegt.

5. Pharmazeutische Zusammensetzung mit verzögerter Freisetzung von Topiramat nach Anspruch 1, wobei der leere Pelletkern einen Partikeldurchmesser von 150 μm bis 1500 μm aufweist.

6. Pharmazeutische Zusammensetzung mit verzögerter Freisetzung von Topiramat nach Anspruch 1, wobei der leere Pelletkern einen Partikeldurchmesser von 300 μm bis 1000 μm aufweist.

7. Pharmazeutische Zusammensetzung mit verzögerter Freisetzung von Topiramat nach Anspruch 1, wobei der leere Pelletkern einen Partikeldurchmesser von 400 μm bis 850 μm aufweist.

8. Pharmazeutische Zusammensetzung mit verzögerter Freisetzung von Topiramat nach Anspruch 1, wobei der leere Pelletkern einen Partikeldurchmesser von 610 μm bis 750 μm aufweist.

9. Pharmazeutische Zusammensetzung mit verzögerter Freisetzung von Topiramat nach Anspruch 1, wobei die Beschichtungsschicht mit verzögerter Freisetzung einen Gewichtsinkrementbereich von 2 % bis 30 % aufweist.

10. Pharmazeutische Zusammensetzung mit verzögerter Freisetzung von Topiramat nach Anspruch 1, wobei die Beschichtungsschicht mit verzögerter Freisetzung einen Gewichtsinkrementbereich von 4 % bis 15 % aufweist.

11. Pharmazeutische Zusammensetzung mit verzögerter Freisetzung von Topiramat nach Anspruch 1, wobei die Beschichtungsschicht mit verzögerter Freisetzung einen Gewichtsinkrementbereich von 5 % bis 10 % aufweist.

12. Pharmazeutische Zusammensetzung mit verzögerter Freisetzung von Topiramat nach Anspruch 1, wobei die Beschichtungsschicht mit verzögerter Freisetzung ein Beschichtungsmaterial mit verzögerter Freisetzung umfasst, bei dem es sich um eines oder mehrere handelt, das bzw. die aus einer Gruppe bestehend aus Ethylcellulose, Eudragit NE 30D, Eudragit RS 30D, Eudragit RL 30D ausgewählt ist bzw. sind.

13. Pharmazeutische Zusammensetzung mit verzögerter Freisetzung von Topiramat nach Anspruch 1 oder 12, wobei die Beschichtungsschicht mit verzögerter Freisetzung Ethylcellulose und PVPK30 umfasst.

14. Pharmazeutische Zusammensetzung mit verzögerter Freisetzung von Topiramat nach Anspruch 13, wobei das Verwendungsmengenverhältnis von Ethylcellulose zu PVPK30 1:0,20 bis 1:0,45 beträgt.

15. Pharmazeutische Zusammensetzung mit verzögerter Freisetzung von Topiramat nach Anspruch 13, wobei das Verwendungsmengenverhältnis von Ethylcellulose zu PVPK30 1:0,25 bis 1:0,40 beträgt.

16. Pharmazeutische Zusammensetzung mit verzögerter Freisetzung von Topiramat nach Anspruch 13, wobei das Verwendungsmengenverhältnis von Ethylcellulose zu PVPK30 1:0,3 bis 1:0,35 beträgt.

**17.** Verfahren zum Herstellen der pharmazeutischen Zusammensetzung mit verzögerter Freisetzung von Topiramat nach einem der Ansprüche 1 bis 17, umfassend die folgenden Schritte:

a) Bereitstellen eines Wirkstoffs der Wirkstoffschicht zum Durchführen einer Wirkstoffbeladung und Beschichten eines leeren Pelletkerns, um ein Wirkstoffbeladungspellet zu erhalten;
b) Unterziehen des Wirkstoffbeladungspellets einer Beschichtung mit verzögerter Freisetzung.

**18.** Verfahren nach Anspruch 17, umfassend die folgenden Schritte:

a) Bereitstellen von Topiramat und anderen Hilfsstoffen der Wirkstoffschicht, Hinzufügen einer geeigneten Menge Lösungsmittel zum Auflösen und Durchführen einer Wirkstoffbeladung und Beschichten eines leeren Pelletkerns, um ein Wirkstoffbeladungspellet zu erhalten;
b) Unterziehen des Wirkstoffbeladungspellets einer Beschichtung mit verzögerter Freisetzung;

**19.** Verfahren nach Anspruch 17, umfassend die folgenden Schritte:

a) Bereitstellen von Topiramat und anderen Hilfsstoffen der Wirkstoffschicht, Hinzufügen einer geeigneten Menge Lösungsmittel zum Auflösen und Durchführen einer Wirkstoffbeladung und Beschichten eines leeren Pelletkerns mit der Wirkstofflösung, um ein Wirkstoffbeladungspellet zu erhalten;
b) Auflösen eines Beschichtungsmaterials mit verzögerter Freisetzung und anderer Hilfsstoffe der Beschichtungsschicht mit verzögerter Freisetzung in einem Lösungsmittel, wobei das Wirkstoffbeladungspellet einer Beschichtung mit verzögerter Freisetzung unterzogen wird;

**20.** Verfahren nach Anspruch 17, umfassend die folgenden Schritte:

a) Bereitstellen von Topiramat und anderen Hilfsstoffen der Wirkstoffschicht, Hinzufügen mit einer geeigneten Menge Lösungsmittel, Erhitzen und Auflösen unter Rühren, Bereitstellen eines leeren Pelletkerns und Platzieren in eine Wirbelbett-Beschichtungspfanne für eine einstufige Granulation, Durchführen einer Wirkstoffbeladung und Beschichten mit der vorstehenden Wirkstofflösung unter Rühren, um ein Wirkstoffbeladungspellet zu erhalten;
b) Auflösen eines Beschichtungsmaterials mit verzögerter Freisetzung und anderer Hilfsstoffe der Beschichtungsschicht mit verzögerter Freisetzung in einem Lösungsmittel, Erhitzen und Auflösen unter Rühren, homogenes Mischen, Sieben durch ein 100-Mesh-Sieb, um eine Beschichtungslösung mit verzögerter Freisetzung zu erhalten;
c) Verwenden des Wirkstoffbeladungspellets, Sprühen der Beschichtungslösung mit verzögerter Freisetzung auf die Oberfläche des Wirkstoffbeladungspellets in einem Wirbelbett, um ein Pellet mit verzögerter Freisetzung von Topiramat zu erhalten.

**21.** Verwendung der pharmazeutischen Zusammensetzung mit verzögerter Freisetzung von Topiramat nach einem der Ansprüche 1 bis 16 bei der Herstellung eines Medikaments zur Prophylaxe und/oder Behandlung und/oder Zusatzbehandlung von Migräne, Epilepsie, Diabetes, Dysneurie, Depression, Psychose, Kopfschmerzen oder Bluthochdruck.

**22.** Pharmazeutischen Zusammensetzung mit verzögerter Freisetzung nach einem der Ansprüche 1 bis 16 zur Verwendung bei der Prophylaxe und/oder Behandlung und/oder Zusatzbehandlung von Migräne, Epilepsie, Diabetes, Dysneurie, Depression, Psychose, Kopfschmerzen oder Bluthochdruck.

## Revendications

**1.** Composition pharmaceutique de topiramate à libération prolongée, qui est un comprimé de topiramate à libération prolongée, et le comprimé de topiramate à libération prolongée comprend un noyau de comprimé exempt de principe actif, une couche de médicament, et une couche d'enrobage à libération prolongée, dans laquelle la couche de médicament est exempte d'agent liant.

**2.** Composition pharmaceutique de topiramate à libération prolongée selon la revendication 1, dans laquelle le topiramate est présent en une quantité de 10 % à 50 % par rapport au poids total de la composition.

3. Composition pharmaceutique de topiramate à libération prolongée selon la revendication 1, dans laquelle le topiramate est présent en une quantité de 15 % à 45 % par rapport au poids total de la composition.

4. Composition pharmaceutique de topiramate à libération prolongée selon la revendication 1, dans laquelle le topiramate est présent en une quantité de 20 % à 40 % par rapport au poids total de la composition.

5. Composition pharmaceutique de topiramate à libération prolongée selon la revendication 1, dans laquelle noyau de comprimé exempt de principe actif a un diamètre des particules de 150 μm à 1 500 μm.

6. Composition pharmaceutique de topiramate à libération prolongée selon la revendication 1, dans laquelle noyau de comprimé exempt de principe actif a un diamètre des particules de 300 μm à 1 000 μm.

7. Composition pharmaceutique de topiramate à libération prolongée selon la revendication 1, dans laquelle noyau de comprimé exempt de principe actif a un diamètre des particules de 400 μm à 850 μm.

8. Composition pharmaceutique de topiramate à libération prolongée selon la revendication 1, dans laquelle noyau de comprimé exempt de principe actif a un diamètre des particules de 610 μm à 750 μm.

9. Composition pharmaceutique de topiramate à libération prolongée selon la revendication 1, dans laquelle la couche d'enrobage à libération prolongée a une plage d'accroissement de poids de 2 % à 30 %.

10. Composition pharmaceutique de topiramate à libération prolongée selon la revendication 1, dans laquelle la couche d'enrobage à libération prolongée a une plage d'accroissement de poids de 4 % à 15 %.

11. Composition pharmaceutique de topiramate à libération prolongée selon la revendication 1, dans laquelle la couche d'enrobage à libération prolongée a une plage d'accroissement de poids de 5 % à 10 %.

12. Composition pharmaceutique de topiramate à libération prolongée selon la revendication 1, dans laquelle la couche d'enrobage à libération prolongée comprend un matériau d'enrobage à libération prolongée, qui est un ou plusieurs sélectionné dans un groupe consistant en l'éthyl cellulose, Eudragit NE 30D, Eudragit RS 30D, Eudragit RL 30D.

13. Composition pharmaceutique de topiramate à libération prolongée selon la revendication 1 ou la revendication 12, dans laquelle la couche d'enrobage à libération prolongée comprend de l'éthyl cellulose et de la PVPK30.

14. Composition pharmaceutique de topiramate à libération prolongée selon la revendication 13, dans laquelle le rapport de la quantité d'usage de l'éthyl cellulose sur la PVPK30 est de 1 : 0,20 à 1 : 0,45.

15. Composition pharmaceutique de topiramate à libération prolongée selon la revendication 13, dans laquelle le rapport de la quantité d'usage de l'éthyl cellulose sur la PVPK30 est de 1 : 0,25 à 1 : 0,40.

16. Composition pharmaceutique de topiramate à libération prolongée selon la revendication 13, dans laquelle le rapport de la quantité d'usage de l'éthyl cellulose sur la PVPK30 est de 1 : 0,3 à 1 : 0,35.

17. Procédé de préparation d'une Composition pharmaceutique de topiramate à libération prolongée selon l'une quelconque des revendications 1 à 17, comprenant les étapes suivantes :

a) fourniture d'un médicament actif de couche de médicament pour réaliser une charge de médicament et un enrobage d'un noyau de comprimé exempt de principe actif pour obtenir un comprimé à charge de médicament ;
b) soumission du comprimé à charge de médicament à un enrobage à libération prolongée.

18. Procédé selon la revendication 17, comprenant les étapes suivantes :

a) fourniture de topiramate et d'autres adjuvants de couche de médicament, ajout d'une quantité adaptée de solvant pour la dissolution, et réalisation d'une charge de médicament et d'un enrobage d'un noyau de comprimé exempt de principe actif pour obtenir un comprimé à charge de médicament ;
b) soumission du comprimé à charge de médicament à un enrobage à libération prolongée.

19. Procédé selon la revendication 17, comprenant les étapes suivantes :

a) fourniture de topiramate et d'autres adjuvants de la couche de médicament, ajout d'une quantité adaptée de solvant pour la dissolution, et réalisation d'une charge de médicament et d'un enrobage d'un noyau de comprimé exempt de principe actif avec la solution de médicament, pour obtenir un comprimé à charge de médicament ;
b) dissolution d'un matériau d'enrobage à libération prolongée et d'autres adjuvants d'une couche d'enrobage à libération prolongée dans un solvant, soumission du comprimé à charge de médicament à un enrobage à libération prolongée.

**20.** Procédé selon la revendication 17, comprenant les étapes suivantes :

a) fourniture du topiramate et d'autres adjuvants d'une couche de médicament, ajout d'une quantité adaptée de solvant, chauffage et dissolution sous agitation, fourniture d'un noyau de comprimé exempt de principe actif et placement dans une turbine d'enrobage à lit fluidisé pour une granulation en une étape, réalisation d'une charge de médicament et d'un enrobage avec la solution de médicament ci-dessus sous agitation, pour obtenir un comprimé à charge de médicament ;
b) dissolution d'un matériau d'enrobage à libération prolongée et des autres adjuvants de couche d'enrobage à libération prolongée dans un solvant, chauffage et dissolution sous agitation, mélange homogène, passage à travers un tamis de 100 mesh, pour obtenir une solution d'enrobage à libération prolongée ;
c) prélèvement du comprimé à charge de médicament, pulvérisation de la solution de revêtement à libération prolongée sur une surface du comprimé à charge de médicament dans un lit fluidisé, pour obtenir un comprimé à libération prolongée de topiramate.

**21.** Utilisation de la composition pharmaceutique de topiramate à libération prolongée selon l'une quelconque des revendications 1 à 16 dans la fabrication d'un médicament pour la prophylaxie et/ou le traitement et/ou le traitement d'appoint de la migraine, de l'épilepsie, du diabète, d'une altération de la fonction nerveuse, de la dépression, de la psychose, des céphalées, ou de l'hypertension.

**22.** Composition pharmaceutique à libération prolongée selon l'une quelconque des revendications 1 à 16 pour son utilisation dans la prophylaxie et/ou le traitement et/ou le traitement d'appoint de la migraine, de l'épilepsie, du diabète, d'une altération de la fonction nerveuse, de la dépression, de la psychose, des céphalées, ou de l'hypertension.

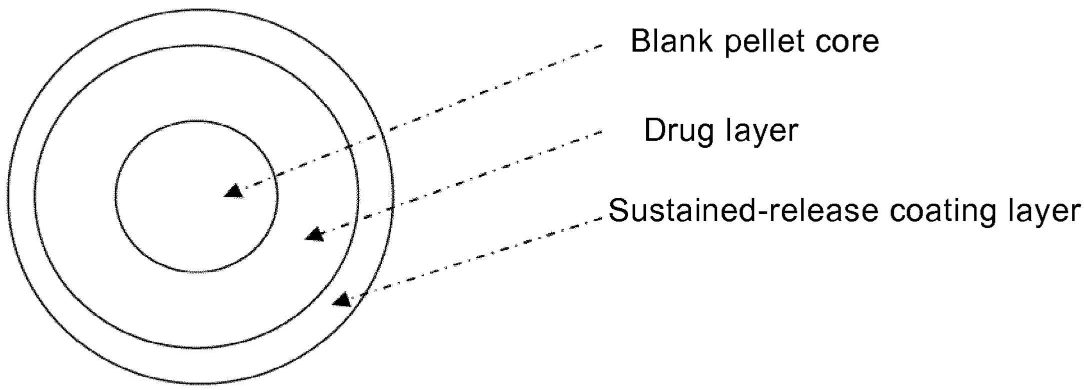
Blank pellet core
Drug layer
Sustained-release coating layer

Fig. 1

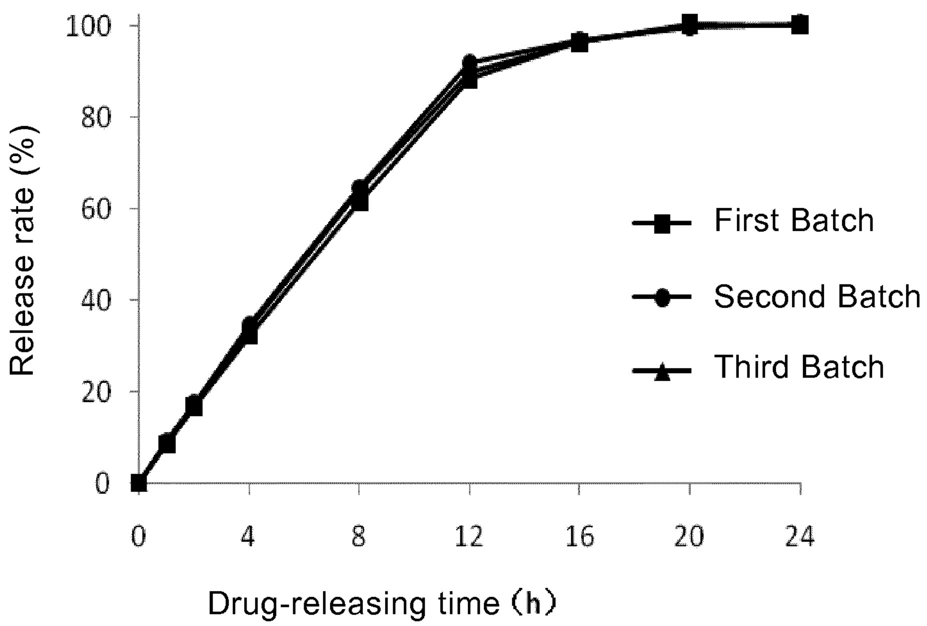
100
80
60
40
20
0
Release rate (%)
0
4
8
12
16
20
24
Drug-releasing time (h)
First Batch
Second Batch
Third Batch

Fig. 2

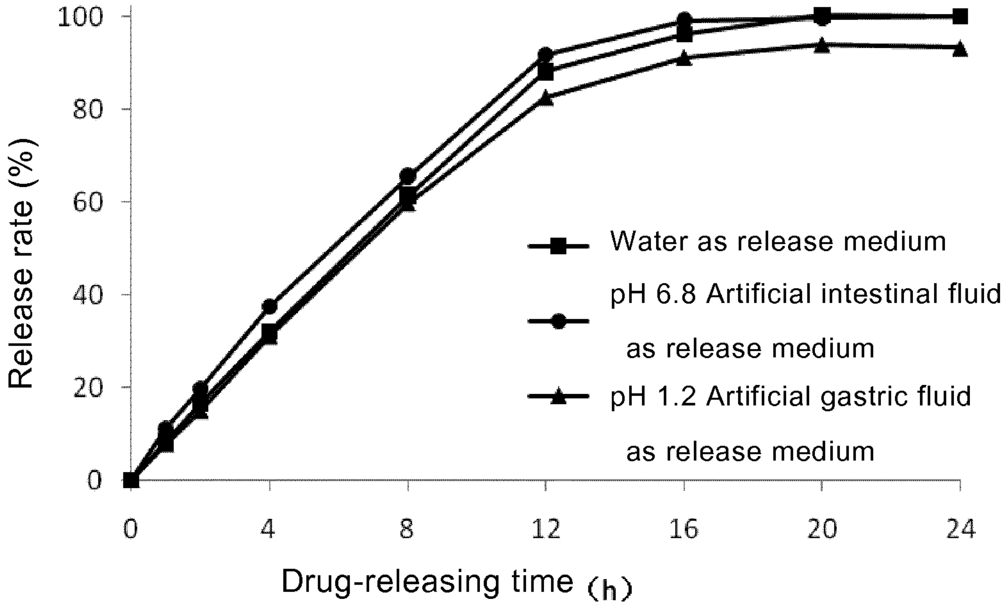
100
80
60
40
20
0
Release rate (%)
0
4
8
12
16
20
24
Drug-releasing time (h)
Water as release medium
pH 6.8 Artificial intestinal fluid as release medium
pH 1.2 Artificial gastric fluid as release medium

Fig. 3

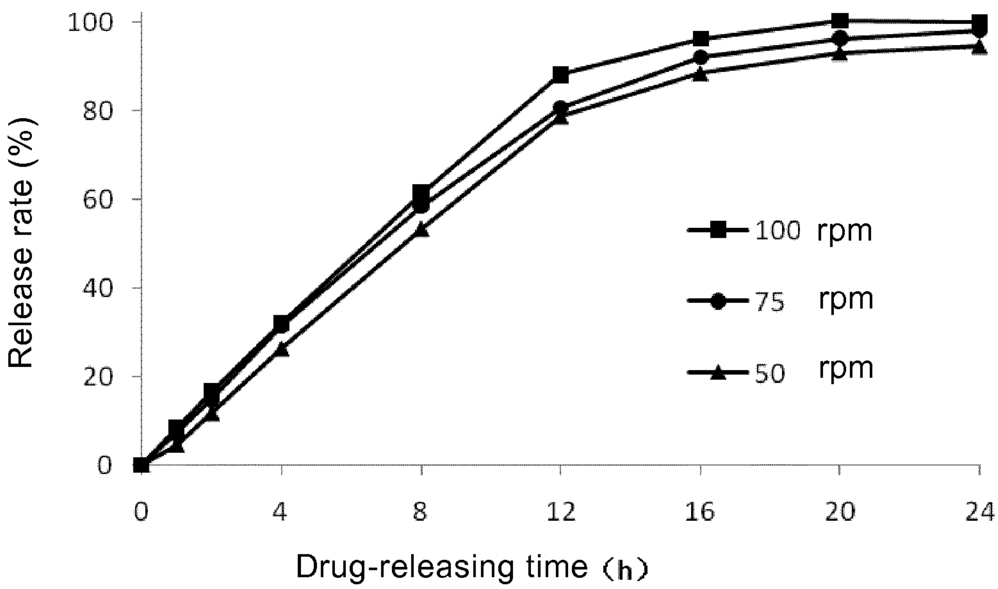
100
80
60
40
20
0
Release rate (%)
0
4
8
12
16
20
24
Drug-releasing time (h)
100 rpm
75 rpm
50 rpm

Fig. 4

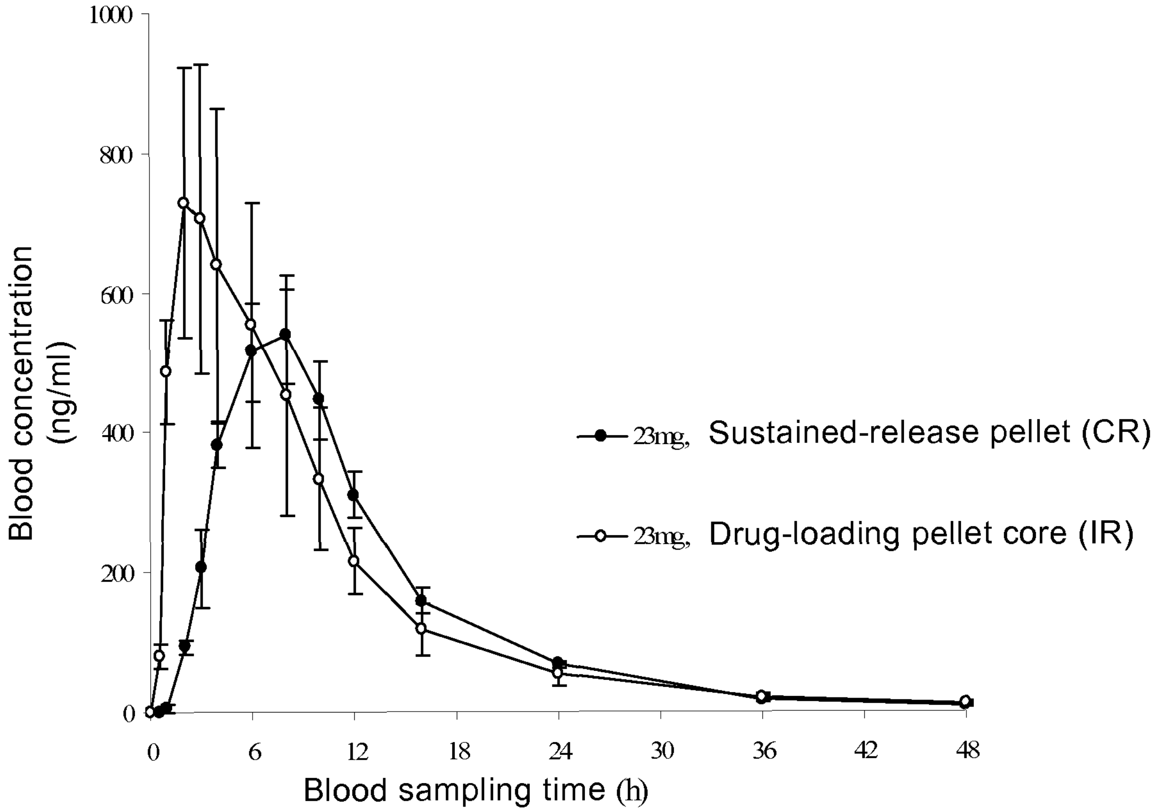
1000
800
600
400
200
0
Blood concentration
(ng/ml)
0
6
12
18
24
30
36
42
48
Blood sampling time (h)
23mg, Sustained-release pellet (CR)
23mg, Drug-loading pellet core (IR)

Fig. 5

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- US 7109174 B2
- US 6362220 B1
- US 6908902 B2
- US 6627653 B2
- US 6620819 B2
- US 6319903 B1
- US 6201010 B1
- CN 1988889 A
- CN 102112126 A
- WO 2008061226 A2
- WO 2011107855 A
- WO 2005079748 A
- WO 2011095973 A
- WO 2007102714 A


### Non-patent literature cited in the description


- **E. FAUGHT et al.** *Neurology,* 1996, vol. 46, 1684-1690
- Physician's Desk Reference. 2002, 2590-2595
- Physician's Desk Reference. 2006, 2538-2447
- Release Rate Measurement (Appendix X D) of Part II of Chinese Pharmacopoeia
- Dissolution Rate Measurement (Appendix X C) of Part II of Chinese Pharmacopoeia
- Appendix V D of Part II of Chinese Pharmacopoeia